# EUROPÄISCHE PATENTSCHRIFT

(11) **EP 0 347 658 B1**
(45) Veröffentlichungstag und Bekanntmachung des Hinweises auf die Patenterteilung: **01.09.1993**
(21) Anmeldenummer: 89110368.1
(22) Anmeldetag: 08.06.1989
(51) Int. Cl.: A61B 17/58

(54) **Knochenplatte, insbesondere Kleinknochenplatte**
Bone plate, especially for small bones
Plaque pour ostéosynthèse, en particulier pour des petits os

(30) Priorität: 18.06.1988 DE 8807909 U
(43) Veröffentlichungstag der Anmeldung: 27.12.1989
(73) Patentinhaber: Howmedica GmbH, D-24232 Schönkirchen (DE)
(72) Erfinder: Luhr, Hans-Georg, Prof. Dr. Dr., D-3400 Göttingen (DE); Harder, Hans Erich, D-2316 Probsteierhagen (DE)
(74) Vertreter: Patentanwälte Hauck, Graalfs, Wehnert, Döring, Siemons

(56) Entgegenhaltungen:
- DE-A- 2 437 752
- FR-A- 2 268 507

## Beschreibung

Die Erfindung bezieht sich auf eine Knochenplatte, insbesondere eine Kleinknochenplatte, nach dem Oberbegriff des Anspruchs 1.

Knochenplatten haben bekanntlich die Aufgabe, Fraktursegmente zu stabilisieren und ein Zusammenwachsen des Knochens im Frakturbereich zu ermöglichen. Es ist auch bekannt, Knochenplatten zweiteilig auszubilden, um einer Längenänderung zu folgen. Eine Längenänderung kann beispielsweise durch Kompression der Fragmente erfolgen, die ihrerseits mit Hilfe einer besonderen Spannvorrichtung durchgeführt wird, wie sie zum Beispiel aus der DE-A-29 38 202 oder DE-A-31 34 120 bekanntgeworden ist. Eine Längenänderung kann beispielsweise auch eintreten bei einer Sinterung im Frakturbereich oder auch durch Knochenwachstum bei jugentlichen Patienten. So ist z. B. eine Platte zur Osteosynthese bekannt geworden, die in ihrem einen Längsabschnitt Kreislöcher und in dem anderen Langlöcher aufweist. Die Platte wird wie bekannte andere Knochenplatten mit Schrauben zu beiden Seiten des Bruches befestigt, die jedoch über dem Abschnitt mit den Langlöchern noch zusätzlich durch eine außen angeordnete Schieberplatte geführt werden. Mit dieser Schieberplatte läßt sich die Knochenplatte an die Relativverschiebungen der Fraktursegmente anpassen. Weiterhin ist zum Beispiel bekannt, zwei teleskopisch ineinandergeführte Knochenplatten mit gezahnten Abschnitten zu versehen, um eine gewünschte Plattenlänge einzustellen und zu fixieren. Zum Spannen von Knochenfragmenten ist aus der DE-A-31 34 120 auch bekanntgeworden, zwischen den zusammenwirkenden Plattenteilen ein Winkelgetriebe anzuordnen. Das Winkelgetriebe ist in einem Gehäuse auf einer ersten Platte angeordnet, während die Ausgangswelle des Winkelgetriebes mit dem zweiten Plattenteil zusammenwirkt, das über eine Knochenschraube mit dem Knochen verbindbar ist, wobei die Knochenschraube durch eine längliche Öffnung des ersten Plattenteils hindurchgeführt ist. Eine derartige Spannvorrichtung mag für Knochenbrüche der Gliedmaßen verwendbar sein, bei kleineren Abmessungen der Knochenplatte, insbesondere bei Anwendungen, in denen nur eine geringe Weichteildeckung vorhanden ist, wie zum Beispiel im Gesichtsbereich oder dergleichen, ist die bekannte Vorrichtung nicht geeignet.

Aus der DE-A-35 04 616 ist auch bekannt, eine Klemmvorrichtung mittels einer Schraubspindel relativ zu einer schraubspindelfesten Klemmvorrichtung zu verstellen. Eine derartige Vorrichtung wird jedoch extern verwendet, kann daher zum Beispiel im Gesichtsbereich nicht eingesetzt werden.

Zufriedenstellend arbeitende Klein- und Kleinstknochenplatten sind zum Beispiel aus dem DE-U-87 06 912 bekanntgeworden. Sie dienen vorwiegend zur Versorgung von Frakturen und Osteotomien des Schädel-Gesichtsskeletts und von Kleinfragmenten anderer Skelettabschnitte. Bei den bekannten Kleinknochenplatten sind sogenannte Schraublochumrandungen mit Stegen verbunden, wobei vorzugsweise zumindest ein Teil der Stege eine Querschnittsfläche aufweist, die gleich oder kleiner ist als das Zweifache der Querschnittsfläche der Schraublochumrandung. Die relativ dünnen Platten sind so ausgelegt, daß sie vom Chirurgen verformt werden können, um sie dem Knochen im zu versorgenden Bereich anzupassen. Es soll dabei sichergestellt werden, daß bei einer Verbiegung der Knochenplatte, insbesondere in ihrer Ebene, die Lochumrandung nicht verbogen wird. Die aus der DE-A-31 34 120 bekanntgewordene Anordnung mag für Knochenbrüche der Gliedmaßen verwendbar sein, bei kleineren Abmessungen der Knochenplatte, insbesondere bei Anwendungen, in denen nur eine geringe Weichteildeckung vorhanden ist, wie zum Beispiel im Gesichtsbereich oder dergleichen, ist die bekannte Vorrichtung nicht geeignet.

Aus der DE-A-24 37 752 ist eine Knochenplatte bekanntgeworden, bei der zwei Plattenteile gegeneinander längsverschieblich gelagert sind und Führungsabschnitte aufweisen, die längsverschieblich in einem Führungsteil geführt sind. Die Führungsabschnitte weisen Zahnungen auf, die mit einem dazwischenliegenden Zahnrad in Eingriff sind. Die Führungsabschnitte werden von einem gehäuseähnlichen Führungsteil geführt.

Der Erfindung liegt die Aufgabe zugrunde, eine Knochenplatte zu schaffen, die auch bei kleinen Knochenplatten exakt geführte Extensionen bzw. Kontraktionen ermöglicht.

Diese Aufgabe wird durch die Merkmale des Patentanspruchs 1 gelöst.

Bei der Erfindung weisen die Führungsabschnitte zwei parallele Schenkel auf, die teleskopisch ineinandergesteckt sind, wobei die jeweils äußere Seite der äußeren Schenkel von der zugekehrten Innenwand des Führungsgehäuses geführt sind. Auf diese Weise findet eine wirksame Abstützung der Führungsteile der Plattenteile innerhalb des Führungsgehäuses statt, durch die sichergestellt ist, daß bei einer Verstellung aufgrund der einseitig angreifenden Zug- oder Druckkraft eine Verklemmung nicht erfolgt.

Die erfindungsgemäße Knochenplatte läßt sich sehr klein und flach bauen, wobei das Führungsteil vorzugsweise eine Breite von 10 mm hat. Seine Dicke kann zum Beispiel 3 mm betragen. Die Knochenplatte selbst hat zum Beispiel eine Dicke von nur 0,7 mm.

Die erfindungsgemäße Knochenplatte kann intern auch dort eingesetzt werden, wo geringe Weichteildeckung vorhanden ist. Das Führungsteil kann so ausgebildet sein, daß der Führungskanal im Gehäuse nur so tief bemessen ist, daß das Zahnrad aufgenommen werden kann bzw. die Zahnabschnitte der Plattenteile Platz finden. Als Angriffsmittel für ein Drehwerkzeug kann beispielsweise ein Innensechskant in der Zahnradwelle gewählt werden. In jedem Fall läßt sich in relativ kurzen Zeitabständen eine Verstellung der neuerungsgemäßen Knochenplatte vornehmen, ohne daß hierdurch der Patient merklich belastet wird. Vorzugsweise ist die erfindungsgemäße Knochenplatte für eine Extension, zum Beispiel bei Osteotomien, mit Vorteil einsetzbar.

In vielen Fällen ist jedoch ein großer Stellweg im Verhältnis zur Umdrehung des Zahnrads nicht erwünscht, im Gegenteil, einem kleinen Verstellweg soll ein relativ großer Drehwinkel entsprechen. Daher sieht eine Ausgestaltung der Erfindung vor, daß mindestens ein weiteres Zahnrad drehbar im Führungsteil gelagert ist, das mit dem ersten Zahnrad in Eingriff steht und einen kleineren Durchmesser aufweist, wobei das zweite Zahnrad Angriffsmittel für ein Drehwerkzeug aufweist. Das zweite Zahnrad kann zum Beispiel ein verhältnismäßig kleines Ritzel sein, so daß eine entsprechende Untersetzung gebildet wird. Theoretisch ist auch möglich, mehr als ein weiteres Zahnrad zu verwenden, beispielsweise auch ein Planetengetriebe, um die gewünschte Untersetzung zu erhalten.

Das Führungsteil bzw. das Führungsgehäuse ist nach einer weiteren Ausgestaltung der Erfindung mit einer Markierung versehen, mit der eine Marke am Zahnrad bzw. an der Zahnradwelle zusammenwirkt, um den Verdrehungswinkel und damit den Verstellweg, zum Beispiel bei einer Extension, anzuzeigen.

Damit bei einer Extension oder einer Kontraktion von außen einwirkende Kräfte nicht zu einer Rückstellung führen, müssen geeignete Feststellmittel vorgesehen werden. So kann zum Beispiel eine Feststellschraube vorgesehen werden, welche die Plattenteile gegeneinander sichert. Alternativ kann die Zahnradwelle oder das Zahnrad selbst mit Hilfe einer Feststellschraube in der jeweiligen Position fixiert werden.

Eine weitere Möglichkeit der Feststellung des Zahnrads besteht erfindungsgemäß darin, daß die Zahnradwelle ein Außengewinde aufweist, auf das eine Kontermutter geschraubt ist, die sich gegen die zugeordnete Fläche des Führungsteils bzw. Führungsgehäuses anlegt. Diese Mutter kann zum Beispiel von einer Art Hülse gebildet sein, die zum Beispiel Sechskantflächen auf der Außenseite trägt. Zum Lösen der Feststellmutter bzw. zum Verdrehen des Zahnrads sind daher zwei verschiedene Schlüsselflächen erforderlich. Es kann hierfür jedoch ein kombiniertes Werkzeug verwendet werden, bei dem zum Beispiel die Schlüsselflächen für den Innensechskant der Zahnradwelle an einem Stab geformt sind, der teleskopisch in einer Hülse verschiebbar ist, die einen Kopf mit Innenschlüsselflächen aufweist. Beim Lösen bzw. Anziehen der Kontermutter wird die Schlüsselhülse etwas über den Schlüsselstab hinausgeschoben, damit er mit der Kontermutter in Eingriff treten kann. Hierbei kann der Schlüsselstab bereits in den Innensechskant der Zahnradwelle eingesteckt sein. Nachdem die Kontermutter gelöst ist, kann daher mit dem Schlüsselstab eine entsprechende Verdrehung vorgenommen werden.

Da es zweckmäßig ist, den Drehwinkel zu kennen, da dieser dem Verstellweg proportional ist, kann eine Skala an zum Beispiel der Werkzeughülse vorgesehen werden, die zum Beispiel bei der Verdrehung des Zahnrads festgehalten wird, während eine Markierung an dem Schlüsselstab sich gegenüber der Skala bewegt, so daß auf diese Weise der Verdrehwinkel abgelesen werden kann.

Die Erfindung wird nachfolgend anhand von Zeichnungen näher erläutert.
- Fig. 1: zeigt vergrößert eine Draufsicht auf die Knochenplatte nach der Erfindung teilweise im Schnitt.
- Fig. 2: zeigt einen Schnitt durch die Knochenplatte nach Fig. 1 entlang der Linie 2-2.
- Fig. 3: zeigt einen Schnitt durch die Knochenplatte nach Fig. 1 entlang der Linie 3-3.
- Fig. 4: zeigt teilweise im Schnitt ein Werkzeug zur Verstellung der Knochenplatte nach den Figuren 1 bis 3.
- Fig. 5: zeigt verschiedene Darstellungen des Zahnrads mit einer Feststellmutter.

Die Zeichnungen sind nur schemahaft und nicht maßstäblich.

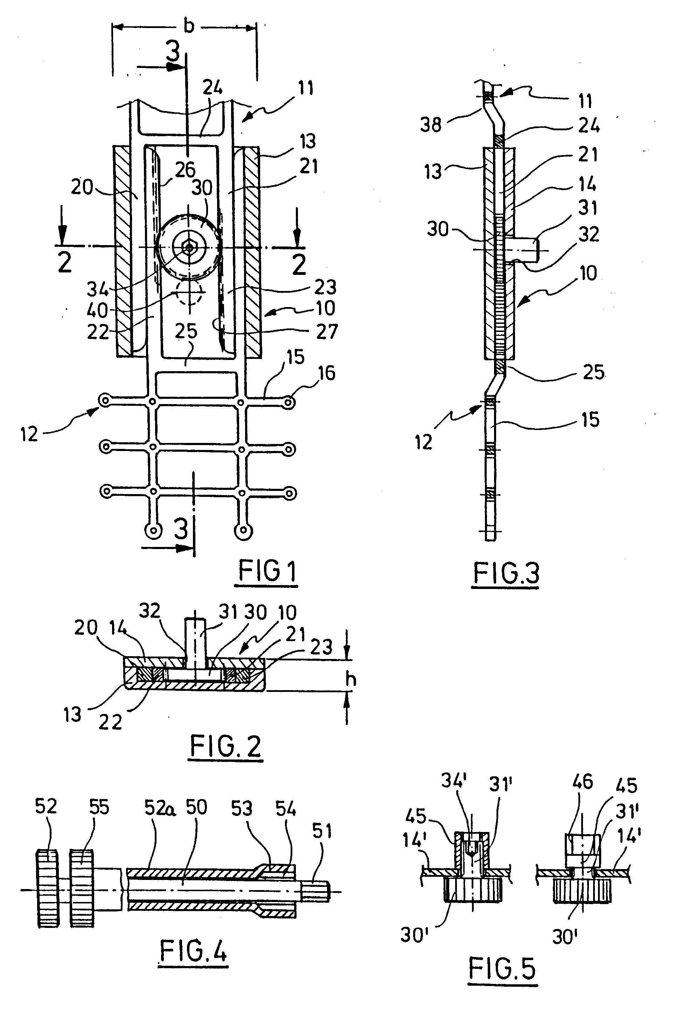

Die in den Figuren 1 bis 3 gezeigte Knochenplatte weist ein Gehäuse 10 auf, in dem auf noch zu beschreibende Weise Plattenteile 11 und 12 geführt sind. Das Gehäuse 10 weist ein im Querschnitt U-förmiges Bodenteil 13 und ein plattenförmiges Deckelteil 14 auf, die beispielsweise durch Verlöten, Verschweißen, Verschrauben oder dergleichen miteinander verbindbar sind. Die Höhe h des Gehäuses beträgt zum Beispiel etwa 3 mm. Seine Breite b beträgt zum Beispiel etwa 10 mm.

Der außerhalb des Gehäuses 10 liegende Bereich der Plattenteile 11, 12 ist herkömmlich aufgebaut. Er besteht aus streifenförmigen Stegen 15, die Lochumrandungen 16 miteinander verbinden, wobei die Lochumrandungen 16 für die Aufnahme von kleinen Knochenschrauben ausgelegt sind. Die Stege 15 sind so bemessen, daß die Knochenplatte durch Verformung von Hand an die Oberflächenkontur des Knochens angepaßt werden kann. Der mit dem Gehäuse 10 zusammenwirkende Abschnitt der Plattenteile 11, 12 ist U-förmig mit Schenkein 20, 21 bzw. 22, 23. Die Schenkel 20, 21 bzw. 22, 23 sind durch Querstege 24 bzw. 25 miteinander verbunden. Jeweils ein Schenkel 20 bzw. 23 der Plattenteile 11, 12 liegt gegen die Gehäuseinnenwand an, wie sich aus den Figuren 1 und 2 ergibt. Auf der Innenseite der Schenkel 20, 23 liegen die anderen beiden Schenkel 22 bzw. 21 an. Diese sind als Zahnstange mit einer Zahnung 26 bzw. 27 ausgebildet.

Zwischen dem Boden des Bodenteils 13 und dem Deckel 14 des Gehäuses ist ein Zahnrad 30 angeordnet, das mittels eines Wellenstumpfs 31 in einer Öffnung 32 des Deckelteils 14 drehbar gelagert ist. Die Breite des Zahnrads 30 entspricht dem Abstand zwischen den Boden des Bodenteils 13 und dem Deckelteil 14. Der Wellenstumpf 31 ist, wie aus Fig. 1 hervorgeht, mit einen Innensechskant 34 versehen. Das Zahnrad 30 wirkt auf diametral gegenüberliegenden Seiten mit der Zahnung 26, 27 der Schenkel 22, 21 zusammen. In der in Fig. 1 dargestellten Position der Plattenteile 11, 12 sind diese am weitesten ineinandergeschoben. Wird das Zahnrad 30 entgegengesetzt dem Uhrzeigersinn gedreht, werden die Plattenteile 11, 12 voneinander fort verstellt, wobei aufgrund der doppelten Verstellung der Plattenteile 11, 12 ein doppelter Verstellweg erhalten wird gegenüber einer Verstellung, bei der nur eine Zahnung mit dem Zahnrad 30 zusammenwirkt.

Nicht gezeigte Feststellmittel, beispielsweise eine Feststellschraube, die mit den Schenkeln der Führungsabschnitte der Plattenteile 11, 12 oder mit dem Zahnrad 30 bzw. dem Zapfen 31 zusammenwirkt, sorgen für eine Fixierung der Plattenteile in der jeweils eingestellten Position.

Wie aus Fig. 3 zu erkennen, sind die außen liegenden Plattenabschnitte in der Ebene gegenüber den Führungsabschnitten innerhalb des Gehäuses 10 versetzt. Dies wird durch eine Kröpfung bewirkt, wie in Fig. 3 durch 38 angedeutet. Dadurch gelangt die Unterseite der außen liegenden Plattenteile in annähernd die gleiche Ebene wie die Unterseite des Gehäuses 10.

Im dargestellten Ausführungsbeispiel ist gezeigt, daß die Querstege 24, 25 bzw. die Schenkel der Führungsabschnitte über zwei Stege mit den außen liegenden Plattenabschnitten verbunden sind. Es versteht sich, daß auch andere Anwendungsmöglichkeiten gewählt werden können.

Ist das Übersetzungsverhältnis zwischen Zahnrad 30 und den Zahnstangen 21, 22 zu groß, kann zum Beispiel ein weiteres Ritzel vorgesehen werden, wie gestrichelt bei 40 in Fig. 1 angedeutet. In diesem Fall wird die Zahnradwelle 31 nicht dem Zahnrad 30, sondern dem Ritzel 40 zugeordnet. Auf diese Weise wird eine entsprechende Untersetzung erreicht, so daß bei einem verhältnismäßig großen Verdrehwinkel nur ein kleiner Verstellweg der Platten 11, 12 erreicht wird.

In Fig. 5 ist ein Zahnrad 30' gezeigt, das dem Zahnrad 30 nach den Figuren 1 bis 3 entspricht. Seine Welle 31' weist ein Außengewinde auf, auf das eine Gewindehülse 45 geschraubt ist. Die Gewindehülse 45 weist einen Außensechskant 46 auf. Die Welle 31' weist einen Innensechskant 34' auf. Wie ohne weiteres zu erkennen, dient die Feststellhülse 45 dazu, die Welle 31' und damit das Zahnrad 30' in einer vorgegebenen Position zu fixieren, wenn die Feststellhülse 45 gegen die Außenseite des Deckenabschnitts 14' des nicht weiter gezeigten Führungsgehäuses anliegt. Wird die Feststellhülse 45 gelöst, kann das Zahnrad 30' mit Hilfe eines entsprechenden Werkzeugs verdreht werden. Ein derartiges Werkzeug ist beispielshalber in Fig. 4 gezeigt. Ein Schlüsselstab 50 weist an einem Ende Sechskantflächen 51 auf, die zum Beispiel mit dem Innensechskant 34 oder 34' in Eingriff bringbar sind. Am anderen Ende des Schlüsselstabs 50 ist ein Rändelknopf 52 angebracht. Auf dem Schlüsselstab 50 ist verschiebbar eine Schlüsselhülse 52a gelagert, die an einem Ende einen Schlüsselkopf 53 mit inneren Sechskantschlüsselflächen 54 und am anderen Ende einen Rändelknopf 55 aufweist. Der Schlüsselkopf 53 kann zum Beispiel mit dem Außensechskant 46 der Feststellhülse 45 zusammenwirken. Mit dem Kombinationswerkzeug nach Fig. 4 kann eine Verstellung des Zahnrads 30' und eine Betätigung der Verstellhülse 45 nacheinander erfolgen. Ferner kann die Rändelmutter 55 mit einer nicht gezeigten Skala versehen sein, der eine nicht gezeigte Marke am Rändelkopf 52 zugeordnet ist, um das Ausmaß der Verdrehung des Zahnrads 30' in Winkelgraden bzw. in einem linearen Verstellweg der Platten 11, 12 festzustellen.

## Patentansprüche

1. Knochenplatte, insbesondere Kleinknochenplatte,mit zwei Plattenteilen (11, 12), die gegeneinander längsverschieblich gelagert sind und Löcher (16) für die Aufnahme von Knochenschrauben und einander zugekehrte Führungsabschnitte (20, 23; 21, 22) aufweisen, die längsverschieblich in einem Führungsteil (10) geführt sind, wobei die Führungsabschnitte eine Zahnung (26, 27) aufweisen und im Führungsteil (10) ein Zahnrad (30) drehbar gelagert ist, das an diametral gegenüberliegenden Punkten mit den Zahnungen (26, 27) in Eingriff steht und Angriffsmittel (34) für ein Drehwerkzeug aufweist, dadurch gekennzeichnet, daß die Führungsabschnitte zwei parallele Schenkel (20, 21 bzw. 22, 23) aufweisen und die Schenkel beider Führungsabschnitte teleskopisch ineinandergesteckt sind, wobei die jeweils äußere Seite der äußeren Schenkel (20, 23) von der zugekehrten Innenwand des Führungsteils (10) geführt sind und die inneren Seiten der inneren Schenkel (21, 22) die Zahnung aufweisen.

2. Knochenplatte nach Anspruch 1, dadurch gekennzeichnet, daß das Führungsteil (10) als flaches Gehäuse ausgebildet ist.

3. Knochenplatte nach Anspruch 2, dadurch gekennzeichnet, daß das Gehäuse einen kanalförmigen Gehäuseabschnitt (13) aufweist, der durch einen plattenförmigen Gehäuseabschnitt (14) abgedeckt ist.

4. Knochenplatte nach einem der Ansprüche 1 bis 3, dadurch gekennzeichnet, daß die Plattenteile (11, 12) nahe dem Führungsteil (10) gekröpft sind (38) dergestalt, daß ihre Unterseite annähernd mit der Unterseite des Führungsteils (10) ausgerichtet ist.

5. Knochenplatte nach einem der Ansprüche 1 bis 4, dadurch gekennzeichnet, daß mindestens ein weiteres Zahnrad (40) drehbar im Führungsteil (10) gelagert ist, das mit dem ersten Zahnrad (30) in Eingriff steht und einen kleineren Durchmesser besitzt und daß das zweite Zahnrad (40) Angriffsmittel für ein Drehwerkzeug aufweist.

6. Knochenplatte nach einem der Ansprüche 1 bis 5, dadurch gekennzeichnet, daß das Zahnrad (30) einen Innensechskant (34) aufweist.

7. Knochenplatte nach einem der Ansprüche 1 bis 6, dadurch gekennzeichnet, daß dem Zahnrad (30) ein Feststellglied zugeordnet ist.

8. Knochenplatte nach einem der Ansprüche 1 bis 7, dadurch gekennzeichnet, daß eine mit dem Zahnrad (30') verbundene Welle (31') ein Außengewinde aufweist, auf das eine Kontermutter (45) geschraubt ist, die gegen die zugeordnete Fläche des Führungsteils (14') schraubbar ist.

9. Knochenplatte nach Anspruch 8, dadurch gekennzeichnet, daß eine hülsenartige Kontermutter (45) vorgesehen ist mit Drehangriffsflächen (46) für ein Werkzeug an der Außenseite.

10. Knochenplatte nach einem der Ansprüche 6 bis 9, gekennzeichnet durch ein Werkzeug für die Zahnradwelle (31') und die Kontermutter (45) derart, daß ein hülsenförmiger erster Schlüssel (52a) für die Kontermutter (45) gleitend auf dem stabförmigen zweiten Schlüssel (50) für den Innensechskant (34') verschiebbar, jedoch feststellbar ist.

11. Knochenplatte nach einem der Ansprüche 1 bis 10, dadurch gekennzeichnet, daß das Führungsteil (10) an der Außenseite eine Markierung trägt und das Zahnrad (30) eine der Markierung zugeordnete Marke aufweist.

12. Knochenplatte nach Anspruch 8, dadurch gekennzeichnet, daß der erste Schlüssel (52a) eine Skala aufweist und der zweite (50) eine Marke trägt, die mit der Skala zusammenwirkt.

## Claims

1. Bone plate, in particular for small bones, comprising a pair of plate members (11, 12) which are longitudinally slidably mounted with respect to each other, including holes (16) for receiving bone screws and guide portions (20, 23; 21, 22) adjacent to each other which guide portions are longitudinally slidably guided in a guide member (10), wherein the guide portions include teeth (26, 27), further comprising a gear wheel (30) rotatably mounted in the guide member (10), diametrically opposed points of the gear wheel meshing with said teeth (26, 27) and said gear wheel including tool engaging means (34), characterized in that the guide portions include a pair of parallel legs (20, 21 or 22, 23), the legs of both guide portions being telescopically stacked within each other, wherein the outer side each of the outer leg (20, 23) is guided by the adjacent inner wall of the guide member (10), and the inner sides of the inner legs (21, 22) include the teeth.

2. The bone plate of claim 1, characterized in that the guide member (10) is formed to be a flat casing.

3. The bone plate of claim 2, characterized in that the casing includes a channel-shaped casing portion (13) which is covered by a plate-shaped casing portion (14).

4. The bone plate of one of claims 1 to 3, characterized in that the plate members (11, 12) are formed to be offset (38) adjacent the guide member (10) such that their lower sides are approximately aligned with the lower side of the guide member (10).

5. The bone plate of one of claims 1 to 4, characterized in that at least a further gear wheel (40) is rotatably mounted in the guide member (10) which gear wheel meshes with the first gear wheel (30), said gear wheel having a smaller diameter, and that the second gear wheel (40) includes tool engaging means.

6. The bone plate of one of claims 1 to 5, characterized in that the gear wheel (30) includes a hexagon socket (34).

7. The bone plate of one of claims 1 to 6, characterized in that a locking means is associated to the gear wheel (30).

8. The bone plate of one of claims 1 to 7, characterized in that a shaft (31') connected to the gear wheel (30') includes an outer thread onto which a counter nut (45) is screwed which may be turned with respect to the associated face of the guide member (40').

9. The bone plate of claim 8, characterized in that a sleeve-like counter nut (45) is provided having outer rotating tool engaging faces (46).

10. The bone plate of one of claims 6 to 9, characterized by a tool for the gear wheel shaft (31') and the counter nut (45) such that a sleeve-like first tool (52a) for the counter nut (45) is slidably, but lockably mounted on a rod-like second tool (50) for the hexagon socket (34').

11. The bone plate of one of claims 1 to 10, characterized in that the outer face of the guide member (10) includes a marking and that the gear wheel (30) includes an index associated to the marking.

12. A bone plate of claim 8, characterized in that the first tool (52a) includes a scale and the second tool (50) includes an index cooperating with said scale.

## Revendications

1. Plaquette pour la chirurgie des os, notamment plaquette pour la chirurgie des petits os, comportant deux parties (11,12), qui sont montées de manière à être déplaçables longitudinalement l'une par rapport à l'autre et comportent des trous (16) destinés à loger des vis à os et des sections de guidage (20,23;21,22) se faisant face et qui sont guidées en déplacement longitudinal dans une pièce de guidage (10), les sections de guidage comportant une denture (26,27), tandis que, dans la pièce de guidage (10), est monté rotatif un pignon (30), qui engrène avec les dentures (26,27) en des points diamétralement opposés, et comporte un moyen d'attaque (34) pour un outil rotatif, caractérisée en ce que les sections de guidage possèdent deux branches parallèles (20,21 ou 22,23) et les branches des deux sections de guidage sont emboîtées l'une dans l'autre de façon télescopique, les côtés extérieurs respectifs des branches extérieures (20,23) étant guidés par la paroi intérieure, qui leur fait face, de la pièce de guidage (10), tandis que les côtés intérieurs des branches intérieures (20,21,22) comportent la denture.

2. Plaquette pour la chirurgie des os selon la revendication 1, caractérisée en ce que la pièce de guidage (10) est réalisée sous la forme d'un boîtier plat.

3. Plaquette pour la chirurgie des os selon la revendication 2, caractérisée en ce que le boîtier présente une portion (13) en forme de canal, qui est recouverte par une portion (14) du boîtier en forme de plaque.

4. Plaquette pour la chirurgie des os selon l'une des revendications 1 à 3, caractérisée en ce que les parties (11,12) de la plaquette sont recourbées (en 38) à proximité de la partie de guidage (10) de telle façon que leur face inférieure est approximativement alignée avec la face inférieure de la pièce de guidage (10).

5. Plaquette pour la chirurgie des os selon l'une des revendications 1 à 4, caractérisée en ce qu'au moins un autre pignon (40), monté rotatif dans la pièce de guidage (10), engrène avec la premier pignon (30) et possède un diamètre plus faible que ce dernier, et en ce que le second pignon (40) comporte des moyens d'attaque pour un outil rotatif.

6. Plaquette pour la chirurgie des os selon l'une des revendications 1 à 5, caractérisée en ce que le pignon (30) possède une cavité à six pans (34).

7. Plaquette pour la chirurgie des os selon l'une des revendications 1 à 6, caractérisée en ce qu'un organe d' immobilisation est associé au pignon (30).

8. Plaquette pour la chirurgie des os selon l'une des revendications 1 à 7, caractérisée en ce qu'un arbre (31') raccordé au pignon (30') possède un filetage extérieur, sur lequel est vissé un contre-écrou (45), qui est vissable contre la surface associée de la partie de guidage (14').

9. Plaquette pour la chirurgie des os selon la revendication 8, caractérisée en ce qu'il est prévu un contre-écrou en forme de douille (45), qui comporte, sur sa face extérieure, des surfaces d'attaque (46), pour son entraînement en rotation par un outil.

10. Plaquette pour la chirurgie des os selon l'une des revendications 6 à 9, caractérisée par un outil pour l'arbre (31') du pignon et le contre-écrou (45) de telle sorte qu'une première clé en forme de douille (52a) pour le contre-écrou (45) peut être déplacée par glissement sur la seconde clé en forme de barre (50) pour la cavité à six pans creux (34'), mais peut être bloquée.

11. Plaquette pour la chirurgie des os selon l'une des revendications 1 à 10, caractérisée en ce que la pièce de guidage (10) porte un moyen de repérage sur sa face extérieure et que le pignon (30) possède un repère associé au moyen de repérage.

12. Plaquette pour la chirurgie des os selon la revendication 8, caractérisée en ce que la première clé (52a) porte une échelle et la seconde clé (50) porte un repère qui coopère avec l'échelle.
